# EUROPEAN PATENT SPECIFICATION

(11) **EP 4 239 078 B1**
(45) Date of publication and mention of the grant of the patent: **14.10.2026**
(21) Application number: 21886365.2
(22) Date of filing: 29.10.2021
(51) Int. Cl.: G01N 33/49

(54) **METHOD FOR DETECTING BLOOD COAGULATION REACTION**
VERFAHREN ZUM NACHWEIS EINER BLUTGERINNUNGSREAKTION
PROCÉDÉ DE DÉTECTION D'UNE RÉACTION DE COAGULATION SANGUINE

(30) Priority: 29.10.2020 JP 2020181453
(43) Date of publication of application: 06.09.2023
(73) Proprietor: Sekisui Medical Co., Ltd., Tokyo 103-0027 (JP)
(72) Inventor: KAWABE, Toshiki, Tokyo 103-0027 (JP); ONISHI, Kengo, Tokyo 103-0027 (JP)
(74) Representative: Wächtershäuser & Hartz Patentanwaltspartnerschaft mbB
(86) International application number: PCT/JP2021/039947
(87) International publication number: WO 2022/092248

(56) References cited:
- WO-A1-2020/158948
- JP-A- 2002 541 431
- JP-A- 2010 217 059
- JP-A- H0 627 115
- JP-A- H06 249 855
- JP-A- H10 123 140
- US-A- 4 720 787
- SHIMOMURA DAIKI, MATSUMOTO TOMOKO, SUGIMOTO KANA, TAKATA TOKIO, KOUNO AYA, SHIMADA MASASHI, MATSUO SHUJI, KAMIOKA MIKIO: "The First-Derivative Curve of the Coagulation Waveform Reveals the Cause of aPTT Prolongation", CLINICAL AND APPLIED THROMBOSIS/HEMOSTASIS, HAGERSTOWN, MD, US, vol. 26, 1 January 2020 (2020-01-01), US , pages 107602962097881, XP055821810, ISSN: 1076-0296, DOI: 10.1177/1076029620978810

## Description

### Field of the Invention

The present invention relates to a method for detecting a blood coagulation reaction.

### Background of the Invention

A blood coagulation test is a test in which a predetermined reagent is added to a blood specimen of a patient and a blood coagulation time or the like is measured in order to diagnose a blood coagulation ability of the patient. Typical examples of the blood coagulation time include a prothrombin time (PT), an activated partial thromboplastin time (APTT), and a thrombin time. An abnormality in the blood coagulation ability causes a prolongation of the coagulation time. Examples of causes of a prolongation of the coagulation time include an effect of a coagulation inhibitor drug, a reduction in a component involved in coagulation, a congenital deficiency of a blood coagulation factor, and an acquired appearance of an autoantibody which inhibits a coagulation reaction.

Recently, automated analyzers which automatically measure blood coagulation reactions are being widely used and enable blood coagulation tests to be readily performed. For example, with an automated analyzer of a certain type, a mixed liquid obtained by adding a reagent to a blood specimen is irradiated with light and a coagulation reaction of the blood specimen is measured based on an obtained change in scattered light quantity. In a normal blood coagulation reaction, the scattered light quantity rises abruptly due to the progress of the coagulation reaction at a time point where a certain amount of time has elapsed from the addition of the reagent and, subsequently, the scattered light quantity saturates and reaches a plateau as the coagulation reaction nears its end, with a time point where the scattered light quantity is maximized being an end time point of the coagulation reaction. A blood coagulation time can be calculated based on such a change in the scattered light quantity over time. On the other hand, photometry data in an analyzer contains various noises attributable to states of the analyzer, a reagent, a specimen, and the like. For example, a subtle increase in scattered light quantity may be measured in an initial stage of a reaction after adding a reagent to a blood specimen and before the scattered light quantity rises due to an original coagulation reaction, and this phenomenon is called an early reaction, a pre-peak, or the like (Patent Literature 1 and Patent Literature 2). Noise in photometry data such as an early reaction can cause an erroneous calculation of a coagulation time.

Methods of eliminating an effect of an early reaction or a pre-peak in automatic measurement of a blood coagulation reaction have been proposed. For example, there is a method for performing a calculation process of a coagulation time by setting thresholds with respect to a measurement time or a measured value in advance and assuming that a reaction after measured time or measured data reaches the threshold to be a true coagulation reaction. In addition, Patent Literature 1 described earlier discloses a method for analyzing a blood coagulation reaction in which an early reaction is detected by monitoring an amount or a rate of optical change in a coagulation reaction at least one check point or in at least one check region between a start of measurement of a coagulation reaction and an end of the reaction. Patent Literature 2 discloses a method for analyzing a blood coagulation in which a step of measuring a scattered light quantity and a step of calculating a time point where the measured scattered light quantity reaches 1/N of a coagulation reaction end point as a coagulation time are repeated until the calculated coagulation time is determined to be normal.

As a method for calculating a coagulation time by an automated analyzer, various methods including a percentage detection method and a differential method are used. For example, in the percentage detection method, after measuring scattered light quantity until the end of a coagulation reaction, a point where X% (for example, 50%) of a light quantity (a maximum scattered light quantity) at the end of the coagulation reaction is reached is detected as the coagulation time. The percentage detection method enables a coagulation time to be accurately calculated even with an abnormal specimen such as a low fibrinogen specimen, a chyle specimen, or a hemolytic specimen. In addition, since the percentage detection method enables a true coagulation reaction which is not noise to be detected by measuring the scattered light quantity until the end of a coagulation reaction, an erroneous calculation of a coagulation time due to the early reaction described earlier can be prevented. On the other hand, in the percentage detection method, in order to enable an end of a coagulation time to be detected with respect to various blood specimens including abnormal specimens with extended coagulation times, the coagulation time is calculated after measuring a single specimen for several minutes. However, normal specimens which make up a majority of blood specimens do not require such a long measurement time.

Patent Literature 3 discloses a method for measuring a blood coagulation time in which scattered light quantity data obtained in real time from an analyzer is subjected to smoothing and origin adjustment and adopted as reference data X, from the reference data, reference integral data Y is calculated by further integrating the reference data and reference ratio data Z is calculated which is a ratio of integrated values between respective adjacent infinitesimal periods of time of the reference data, among time points where the reference ratio data Z assumes a certain reference ratio data value Zs determined in advance, a reference data value Xd at a time point which is subsequent to a peak of the reference ratio data Z and at which the reference integral data Y equals or exceeds a threshold Ys determined in advance is selected, and a period of time from a time point of mixing to a time point corresponding to a value which is 1/N (where N is a certain integer equal to or larger than 1) of Xd is assumed to be the coagulation time.

### [Citation List]

### [Patent Literature]

[Patent Literature 1] JP-A-2003-169700
[Patent Literature 2] JP-A-2010-217059
[Patent Literature 3] JP-A-H6-249855

### Summary of the Invention

The present invention provides a method for detecting a blood coagulation reaction which enables a true coagulation reaction to be detected in real time during measurement of a blood coagulation reaction.

Specifically, the present invention provides the following:
[1] A method for detecting a blood coagulation reaction, comprising:
   1) measuring a blood coagulation reaction of a subject blood specimen and acquiring a first derivative V(i) of a coagulation reaction up to a latest measurement point, where i represents a measurement point or time and satisfies i = k₀ to k, k represents a latest measurement point or time with respect to a latest V(i), and k₀ represents any measurement point or time where k₀ ≤ k,
   2) calculating areas under the curve (AUCs) before the peak (pre-peak AUC) and after the peak (post-peak AUC) of V(i), wherein a top of the peak of V(i) is cVmax(k) which is a maximum value of V(i) up to i=k and
   3) detecting cVmax(k) as Vmax which is a true maximum value of V(i) when the pre-peak AUC and the post-peak AUC are both equal to or higher than a first threshold AUCₜₕ₁ and a period L during which the pre-peak AUC and the post-peak AUC have continued to be a constant value reaches a predetermined length, wherein this result indicates at least that a true coagulation reaction is correctly detected.
[2] The method according to [1], wherein
   the step 2) comprises calculating cVmax(k), cVmaxT(k), AUCₚᵣₑ(k), and AUCₚₒₛₜ(k), where
      cVmax(k) represents a maximum value of V(i) (i = k₀ to k),
      cVmaxT(k) represents a measurement point or time where V(i) = cVmax(k) is satisfied,
      AUCₚᵣₑ(k) represents a pre-peak AUC at i=k which is an AUC of V(i) from i=k₁ to i=cVmaxT(k),
      AUCₚₒₛₜ(k) represents a post-peak AUC at i=k which is an AUC of V(i) from i=cVmaxT(k) to i=k₂,
      k₁ represents a latest measurement point or time among measurement points or times where V(i) ≤ cVmax(k) × Hr% is satisfied prior to cVmaxT(k),
      k₂ represents an earliest measurement point or time among measurement points or times where V(i) ≤ cVmax(k) × Hr% is satisfied after cVmaxT(k),
      0 < Hr < 100, and
   the method comprises
   repeating the steps 1) to 3), assuming that k = k + x (x > 0) when the Vmax is not detected in the step 3).
[3] The method according to [2], wherein
   the step 3) comprises:
   adopting L = L + 1 when both AUCₚᵣₑ(k) and AUCₚₒₛₜ(k) are equal to or larger than AUCₜₕ₁ and, at the same time,
   when AUCₚᵣₑ(k) = AUCₚᵣₑ(k - x) and AUCₚₒₛₜ(k) = AUCₚₒₛₜ(k - x) where x > 0 are satisfied, but otherwise adopting L = 0; and
   detecting cVmax(k) as the Vmax of V(i) when L reaches a predetermined value.
[4] The method according to [2] or [3], wherein 10 ≤ Hr ≤ 70.
[5] The method according to any one of [1] to [4], wherein in the step 3), Vmax is not detected when a measurement point or time which equals cVmax(k) is included in a detection exclusion range.
[6] The method according to any one of [1] to [5], further comprising detecting cVmax(k) as the Vmax of V(i) when the pre-peak AUC at i=k is equal to or larger than the first threshold AUCₜₕ₁ when k exceeds a measurement end point or time without detecting the Vmax.
[7] The method according to any one of [1] to [6], further comprising detecting cVmax(k) as the Vmax of V(i) when both the pre-peak AUC and the post-peak AUC at i=k are equal to or larger than a second threshold AUCₜₕ₂, a measurement point or time at which the pre-peak AUC is maximized is the same as a measurement point or time which equals cVmax(k), and a measurement point or time at which the post-peak AUC is maximized is later than the measurement point or time which equals cVmax(k) when k exceeds a measurement end point or time without detecting the Vmax.
[8] The method according to any one of [1] to [7], further comprising obtaining the coagulation reaction up to the latest measurement point as a reaction P(i).
[9] The method according to [8], further comprising calculating a blood coagulation time of the subject blood specimen based on P(i) or V(i).
[10] The method according to [9], wherein i ≥ k₁ is satisfied.

### Advantageous Effects of Invention

With the method according to the present invention, an erroneous detection of noise such as an early reaction in blood coagulation reaction measurement can be prevented and a true coagulation reaction can be correctly detected in real time during the measurement. With the method according to the present invention, a coagulation time based on a true coagulation reaction can be correctly calculated. In addition, a real-time detection of a true coagulation reaction by the method according to the present invention enables the measurement of a single blood specimen to be stopped once data necessary for calculating a coagulation time of the specimen has been acquired and enables a transition to be made to the measurement of a next blood specimen. Therefore, with the method according to the present invention, a measurement time required for a single blood specimen can be reduced and efficiency of a blood coagulation test can be improved.

### Brief Description of Drawings

[Figure 1] Figure 1 shows an example of measured data of a coagulation reaction.
[Figure 2] Figure 2 is a conceptual diagram explaining cVmax(k), cVmaxT (k), AUCₚᵣₑ(k), and AUCₚₒₛₜ(k).
[Figure 3] Figure 3 shows changes in AUCₚᵣₑ(k) and AUCₚₒₛₜ(k) as a coagulation reaction progresses. A: Progression of coagulation reaction. B: Changes in AUCₚᵣₑ(k) and AUCₚₒₛₜ(k) over time.
[Figure 4] Figure 4 shows a coagulation reaction having an early reaction. A: pre-peak AUC and post-peak AUC of V(i) of an early reaction and a true reaction, and B: enlarged view of the early reaction.
[Figure 5] Figure 5 shows a coagulation reaction having an early reaction and coagulation reactions not having an early reaction.
[Figure 6] Figure 6 is a flow chart explaining an embodiment of procedures of a method according to the present invention.
[Figure 7] Figure 7 shows coagulation reactions of three subject specimens with different coagulation abilities.
[Figure 8] Figure 8 is a conceptual diagram showing a configuration of an automated analyzer for carrying out a method for detecting a blood coagulation reaction according to the present invention.
[Figure 9] Figure 9 shows a relationship between parameters calculated from a coagulation reaction.
[Figure 10] Figure 10 shows a plot of time (Fix) at which Vmax was detected in Example 3 with respect to VmaxT measured in Example 1.
[Figure 11] Figure 11 is a flow chart of procedures of detecting Vmax used in Example 4.
[Figure 12] Figure 12 shows changes over time of parameters calculated from a specimen 1 in Example 4.

### Detailed Description of the Invention

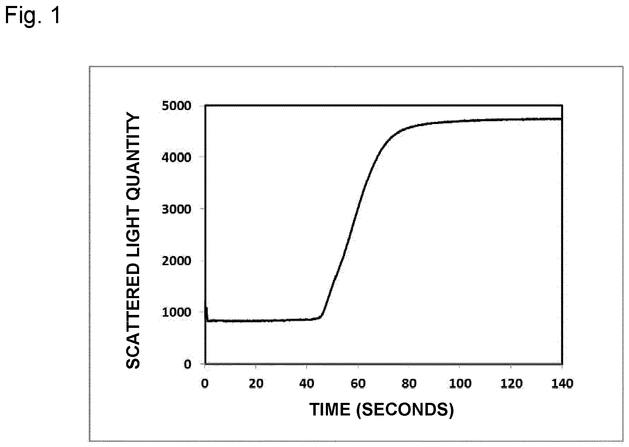

In a blood coagulation test, a predetermined reagent is added to a blood specimen, a subsequent blood coagulation reaction is measured, and a blood coagulation time is calculated from the coagulation reaction. In the present specification, a blood specimen may be simply referred to as a specimen. In the measurement of the blood coagulation reaction, general means such as optical means which measures a scattered light quantity, transmittance, absorbance, or the like, mechanical means which measures a viscosity of blood plasma, or the like is used. A blood coagulation reaction is generally represented by a coagulation reaction curve indicating a change in an amount of a coagulation reaction over time. Although dependent on measuring means, the coagulation reaction curve of a normal specimen without a coagulation abnormality factor basically exhibits a sigmoidal shape. For example, as shown in Figure 1, the coagulation reaction curve of a normal specimen based on a scattered light quantity usually rises abruptly as coagulation progresses at a time point where a certain amount of time has elapsed from the addition of a reagent and, subsequently, reaches a plateau as the coagulation reaction nears its end. On the other hand, the coagulation reaction curve of an abnormal specimen with a coagulation abnormality factor exhibits various shapes dependent on a cause of the abnormality such as a delayed rise time or a gradual rise of the curve.

In the measurement of a blood coagulation time of a specimen, data can be collected until the end of the coagulation reaction or, in other words, until the coagulation reaction curve reaches a plateau, and a coagulation time can be calculated based on the data. For example, in a percentage detection method, if a reaction amount from a start of a reaction to an end of the reaction is taken as 100%, a time until the reaction amount reaches a predetermined value (for example, 50%) can be calculated as a coagulation time. Alternatively, the coagulation time can be calculated based on a rate of change of the coagulation reaction curve such as a peak of a coagulation reaction rate (so-called differential method) or a change over time of an integrated value of a coagulation reaction during a small time slot (refer to Patent Literature 3). However, since the percentage detection method requires that a measurement time per specimen be set sufficiently long in order to enable a scattered light quantity until an end of a coagulation reaction to be measured with respect to various blood specimens including abnormal specimens with extended coagulation times, analysis efficiency is not high. On the other hand, with the latter method, although a coagulation time can be calculated in a shorter period of time since the coagulation time can be calculated before the end of the coagulation reaction, an inaccurate coagulation time may be calculated due to noise.

In coagulation reaction measurement, a coagulation reaction may be detected at a premature time point due to device noise in an initial stage of the measurement or due to initial noise such as an early reaction. Such an erroneous detection of a coagulation reaction may result in a calculation of an inaccurate coagulation time. In order to prevent an erroneous detection of a coagulation reaction at a premature time point, there is a method for performing a calculation process of a coagulation time by setting thresholds with respect to a measurement time or a measured value in advance and assuming that a reaction after measured time or measured data reaches the threshold to be a true coagulation reaction. However, setting a threshold too high may prevent a true coagulation reaction from being detected particularly in abnormal specimens with small coagulation reactions and, on the other hand, setting a low threshold increases a possibility of an erroneous detection of noise.

In blood coagulation reaction measurement, it is desired that a true coagulation reaction be correctly detected and a coagulation time be accurately calculated without erroneously detecting noise such as an early reaction. In addition, with a conventional method for analyzing a blood coagulation reaction in which a coagulation time is calculated after detecting an end of a coagulation reaction, while the coagulation time can be accurately calculated, since a measurement time per specimen is long, analysis efficiency is low. It is more preferable if the measurement time per specimen can be shortened and the analysis efficiency of a blood specimen can be improved while preventing erroneous detection of noise.

### [1. Method for detecting blood coagulation reaction]

The present invention provides a method for detecting a blood coagulation reaction. The method for detecting a blood coagulation reaction according to the present invention (hereinafter, also referred to as a method according to the present invention) typically includes the following:
1) measuring a blood coagulation reaction of a subject blood specimen and acquiring a first derivative V(i) of a coagulation reaction up to a latest measurement point, where i represents a measurement point or time and satisfies i = k₀ to k, k represents a latest measurement point or time with respect to a latest V(i), and k₀ represents any measurement point or time where k₀ ≤ k;
2) calculating areas under the curve (AUCs) before the peak (pre-peak AUC) and after the peak (post-peak AUC) of V(i), wherein a top of the peak of V(i) is cVmax(k) which is a maximum value of V(i) up to i=k; and
3) detecting cVmax(k) as Vmax which is a true maximum value of V(i) when the pre-peak AUC and the post-peak AUC are both equal to or higher than a first threshold AUCₜₕ₁ and a period L during which the pre-peak AUC and the post-peak AUC have continued to be a constant value reaches a predetermined length.

In the method according to the present invention, while measuring a blood coagulation reaction of a subject blood specimen (hereinafter, also referred to as a subject specimen), a true coagulation reaction (hereinafter, also referred to as a true reaction) is detected in real time from time-series data of a coagulation reaction obtained by the measurement. The method according to the present invention enables an erroneous detection of noise such as an early reaction to be prevented and a true reaction to be correctly detected during a blood coagulation reaction measurement. A blood coagulation time of the subject specimen can be calculated based on the obtained true reaction. With the method according to the present invention, a coagulation time can be correctly calculated without being affected by noise such as an early reaction. In addition, according to the present invention, a measurement time can be optimized with respect to various blood specimens including normal specimens and abnormal specimens so that a minimum coagulation reaction measurement time is applied to each calculation of a coagulation time.

Examples of the blood coagulation time which can be calculated in accordance with the present invention include a prothrombin time (PT), an activated partial thromboplastin time (APTT), and a coagulation time in fibrinogen (Fbg) concentration measurement. In the present specification presented below, the method according to the present invention will be described by mainly taking an activated partial thromboplastin time (APTT) as an example of a coagulation time. Any person skilled in the art can modify the method according to the present invention to other coagulation times (for example, a prothrombin time (PT)).

Hereinafter, procedures of the method according to the present invention will be described in detail.

In the method according to the present invention, blood plasma of a subject being tested is preferably used as a subject specimen. An anticoagulant normally used in a coagulation test can be added to the specimen. For example, blood plasma is obtained by collecting blood using a blood collection tube with sodium citrate and subsequently subjecting the collected blood to centrifugal separation.

In a measurement of a blood coagulation reaction, a reagent for coagulation time measurement is added to the subject specimen to start a blood coagulation reaction. A coagulation reaction of a mixed liquid containing the reagent and the subject specimen can be measured. The reagent for coagulation time measurement to be used can be optionally selected in accordance with the purpose of measurement. Reagents for measuring various coagulation times are commercially available (for example, APTT Reagent Coagpia APTT-N manufactured by SEKISUI MEDICAL CO., LTD.). In the measurement of a coagulation reaction, general means such as optical means which measures a scattered light quantity, transmittance, absorbance, or the like, mechanical means which measures a viscosity of blood plasma, or the like may be used. In the present specification presented below, the method according to the present invention will be described using a coagulation reaction measurement based on a scattered light quantity as an example.

While a reaction start time point of a coagulation reaction can be typically defined as a time point where a reagent is mixed with a specimen and the coagulation reaction is started, other timings may be defined as the reaction start time point. A period of time during which the measurement of the coagulation reaction is continued can be, for example, several ten seconds to around eight minutes from the time point at which the reagent is mixed with the specimen. While this measurement time may be up to a time point where a true coagulation reaction of each specimen is detected, the measurement time may be up to a time point when other optional conditions are satisfied or may be an optionally-determined fixed value. During the measurement time, a measurement (photometry in a case where detection is performed optically) of a progress of the coagulation reaction can be repetitively performed at predetermined intervals. For example, measurements may be performed at 0.1-second intervals. A temperature of the mixed liquid during the measurement corresponds to normal conditions such as 30°C or higher and 40°C or lower and, preferably, 35°C or higher and 39°C or lower. In addition, various conditions of measurement can be appropriately set in accordance with the subject specimen, the reagent, the measurement means, and the like.

A series of operations in the coagulation reaction measurement described above can be performed using an automated analyzer. Examples of the automated analyzer include the Automated Coagulation Analyzer CP3000 (manufactured by SEKISUI MEDICAL CO., LTD.). Alternatively, a part of the operations may be performed manually. For example, the subject specimen can be prepared by hand and subsequent operations can be performed by an automated analyzer.

In step 1) of the method according to the present invention, a blood coagulation reaction of a subject specimen is measured and a first derivative V(i) of the coagulation reaction up to a latest measurement point is acquired.

First, due to the coagulation reaction measurement described above, measured data D(i) (a photometric value of a scattered light quantity) is sequentially acquired. In this case, **"i"** represents a measurement point or, in other words, an order of measurement as counted from start of the measurement. Alternatively, "i" represents a time from start of a coagulation reaction (also simply referred to as time). For example, when a measurement (photometry) interval is 0.1 seconds, "i" is represented as time = 0.1 × i.

Next, a reaction P(i) is acquired from the measured data D(i). Since the measured data D(i) contains noise during photometry and fluctuations which appear immediately after start of photometry and which are unrelated to the reaction, the measured value is preferably subjected to smoothing by known methods. In addition, when a coagulation reaction is photometrically measured based on a scattered light quantity, zero point adjustment processing of subtracting a scattered light quantity derived from the mixed specimen liquid prior to the reaction is preferably performed. Any of various known methods related to denoising can be used in the smoothing of measured data. For example, examples of smoothing include filtering and processing in which a difference value is obtained or a derivative value is obtained by an operation such as an intra-section average gradient to be described later and the derivative value is subsequently integrated. In zero point adjustment, for example, smoothed measured data may be adjusted so as to assume a value of 0 at a measurement start time point. Preferably, the measured data D(i) is subjected to smoothing or zero point adjustment to acquire a reaction P(i). More preferably, the measured data D(i) is subjected to smoothing and zero point adjustment to acquire the reaction P(i). The reaction P(i) constitutes a coagulation reaction curve.

A first derivative V(i) thereof is acquired from the obtained reaction P(i). Differential processing of obtaining V(i) from P(i) can be performed by any method such as calculating an intra-section average gradient value. In the calculation of an intra-section average gradient value, a certain number of measurement points before and after each measurement point i such as 2K+1-number of measurement points from i-K to i+K can be used, where K denotes any integer. For example, when K is 2, five measurement points of i-2, i-1, i, i+1, and i+2 can be used. An average gradient value refers to a gradient value when linearly approximating the plurality of measurement points. Conventional methods such as a least-squares method can be used as a method to calculate linear approximation. An average gradient value of the measurement points can be considered a first derivative at the measurement point **i.** The first derivative V(i) constitutes a curve representing a rate of a coagulation reaction.

V(i) used in the method according to the present invention may be a first derivative value calculated from a coagulation reaction P from any measurement point or time to a latest measurement point or time. Therefore, V(i) used in the method according to the present invention can constitute a curve which extends as coagulation reaction measurement progresses. Specifically, in the method according to the present invention, V(i) at i = k₀ to k can be acquired. k represents a latest measurement point or measurement time with respect to a latest V(i). Therefore, k increases as coagulation reaction measurement progresses. Note that the "latest V(i)" refers to V(i) having been most recently calculated using a coagulation reaction P at a latest measurement point or time. A latest measurement point or time among the measurement points or times with respect to V(i) is the "latest measurement point or time with respect to V(i)". For example, if V(i) represents coagulation reaction rate data from 1 second to 100 seconds after measurement, then 100 seconds is the "latest measurement time" with respect to V(i). k₀ represents any measurement point or time prior to k (in other words, k₀ ≤ k). k₀ represents a measurement point or time at which a detection process of a true reaction shown in step 2) is started and is also referred to as a detection start point (or a detection start time) in the present specification. k₀ is a measurement point or a time corresponding to the measurement point subsequent to a point at which acquisition of P(i) due to smoothing of D(i) or acquisition of V(i) due to a differentiation of P(i) is possible and, therefore, the measurement point k₀ is basically 2 or more. For example, when performing an intra-section average gradient method using the five measurement points described above, the measurement point k₀ is 3 or more. In addition, for example, when using 20 measurement points for smoothing or differentiation, the measurement point k₀ is 21 or more.

In an embodiment, k₀ denotes a measurement point or time after a detection exclusion range set in an initial stage of measurement. The detection exclusion range corresponds to a measurement range or a time domain in which a true reaction cannot possibly occur in the initial stage of measurement. Preferably, under standard conditions of an APTT measurement, the detection exclusion range is set as a time domain of around 10 seconds from the start of measurement or a measurement range corresponding to the time range and, in this case, the time k₀ is longer than 10 seconds. This similarly applies to a PT measurement. Under measurement conditions for a Fbg concentration measurement, the detection exclusion range is set as a time domain of around 3 to 4 seconds from the start of measurement or a measurement range corresponding to the time domain and, in this case, the time k₀ is longer than 3 seconds. By setting a detection exclusion range, noise which occurs in an initial stage of measurement can be excluded from the detection process of a true reaction.

In another embodiment, k₀ is a measurement point or time at which V(i) reaches a threshold Vₜₕ for the first time. By setting the threshold Vₜₕ, minute noise which is mixed in a coagulation reaction can be excluded from the detection process of a true reaction.

In step 2) of the method according to the present invention, areas under the curve (AUCs) before the peak (pre-peak AUC) and after the peak (post-peak AUC) of V(i) acquired in step 1) are calculated.

For the calculation of the pre-peak AUC and the post-peak AUC, a peak of V(i) having a maximum value of V(i) up to the measurement point or the time k acquired in step 1) as a peak top is used. The maximum value of V(i) at k will be referred to as cVmax(k) in the present specification. cVmax(k) can fluctuate depending on k. Therefore, the pre-peak AUC and the post-peak AUC may also fluctuate depending on k and, therefore, the pre-peak AUC and the post-peak AUC are respectively expressed as variables AUCₚᵣₑ(k) and AUCₚₒₛₜ(k) of k in the present specification.

Preferably, in step 2), cVmax(k), cVmaxT(k), AUCₚᵣₑ(k), and AUCₚₒₛₜ(k) are calculated.
cVmax(k) represents a maximum value of V(i) up to the measurement point or time k acquired in step 1) or, in other words, the maximum value of V (i) (i = k₀ to k).
cVmaxT(k) represents a measurement point or time where V(i) = cVmax(k) is satisfied.
AUCₚᵣₑ(k) and AUCₚₒₛₜ(k) are, respectively, the pre-peak AUC and the post-peak AUC at k described above. AUCₚᵣₑ(k) represents the AUC of V(i) from k₁ to cVmaxT (k) and AUCₚₒₛₜ(k) represents the AUC of V(i) from cVmaxT(k) to k₂, where k₁ denotes a latest measurement point or time among the measurement points or times at which V(i) ≤ cVmax(k) × Hr% is satisfied before cVmaxT(k), and k₂ denotes an earliest measurement point or time among the measurement points or times that satisfy V(i) ≤ cVmax(k) × Hr% after cVmaxT(k). Therefore, basically, k₁ ≤ k₂ is satisfied, and from a rising edge of the peak to the peak top of V(i), AUCₚₒₛₜ(k) = 0 is satisfied. k₁ and k₂ are, respectively, a start point of the calculation of AUCₚᵣₑ(k) and an end point of the calculation of AUCₚₒₛₜ(k). Hr denotes a height ratio and determines a height of a start point or an end point of V (i) used in the calculations of AUCₚᵣₑ(k) and AUCₚₒₛₜ(k). Hr is set to any value larger than 0 and smaller than 100 (0 <Hr <100) and, preferably, within a range of 10 ≤ Hr ≤ 70.

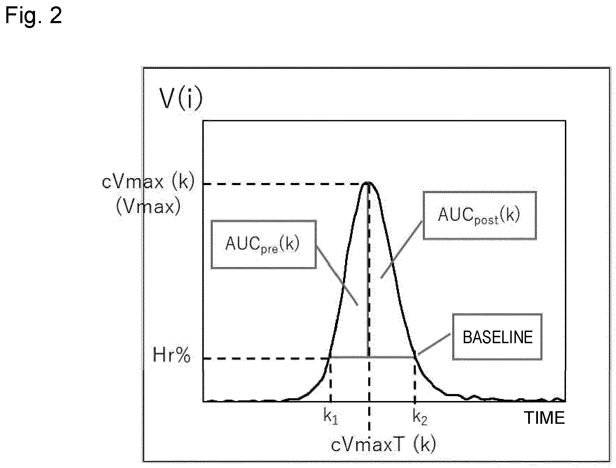

Referring to Figure 2, cVmax(k), cVmaxT(k), AUCₚᵣₑ(k), and AUCₚₒₛₜ(k) will be explained. In Figure 2, a first derivative V(i) of a coagulation reaction curve is plotted against time. In the case of Figure 2, at the measurement point k, the peak top of V (i) is the maximum value cVmax(k), and the time at that point is cVmaxT(k). A line (baseline) indicating cVmax(k) × Hr% is drawn under the curve of V (i). There are two time points (intersection of V(i) and baseline) where V(i) ≥ cVmax(k) × Hr% is satisfied, one of which being k₁ which is present before cVmaxT(k) and the other being k₂ which is present after cVmaxT(k). k₁ and k₂ correspond to a start point and an end point of the baseline, respectively. The AUC prior to the peak of V(i) or, in other words, the AUC from k₁ to cVmaxT(k) is AUCₚᵣₑ(k), and the AUC after the peak of V(i) or, in other words, the AUC from cVmaxT(k) to k₂ is AUCₚₒₛₜ(k) .

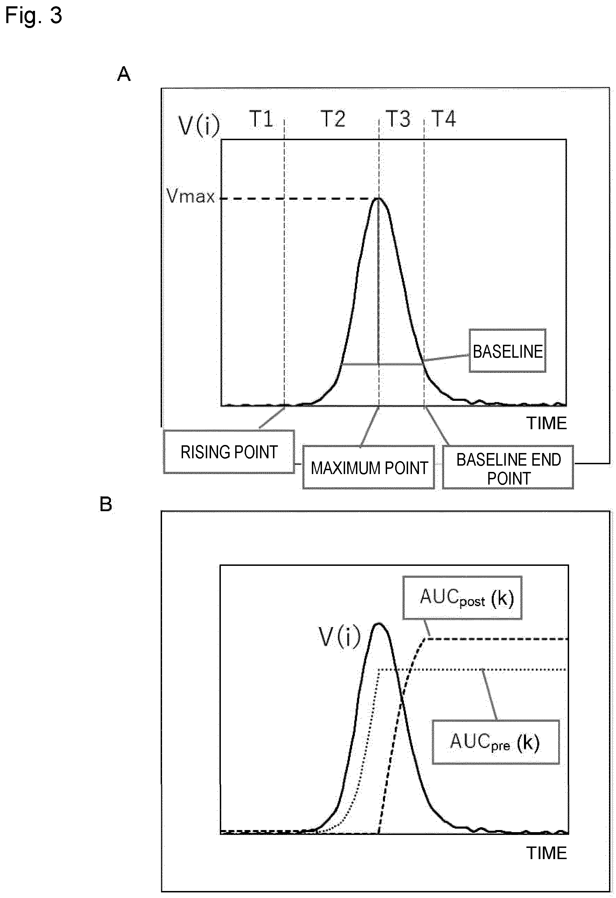

Changes in AUCₚᵣₑ(k) and AUCₚₒₛₜ(k) as a coagulation reaction progresses will be explained with reference to Figure 3. As shown in Figure 3A, V(i) was divided into four sections T1 to T4 described below in a horizontal axis direction (reaction progress). As a baseline, a line representing Hr% of a peak top Vmax of V(i) was set. The maximum point is a time at which V(i) = Vmax is satisfied. i and k represent time.
- T1:: To a rising point of the reaction
- T2:: From the rising point to a maximum point
- T3:: From the maximum point to an end point of the baseline
- T4:: After the end point of the baseline

Table 1 shows behaviors of cVmax(k), AUCₚᵣₑ(k), and AUCₚₒₛₜ(k) at T1 to T4. Before the start of a coagulation reaction indicated by T1, both AUCₚᵣₑ(k) and AUCₚₒₛₜ(k) are 0. In a rising portion of V(i) indicated by T2, cVmax(k) increases with k or, in other words, cVmax(k) = V(k) is satisfied and, therefore, AUCₚᵣₑ(k) also increases with k, but since there is no post-peak region, AUCₚₒₛₜ(k) is 0. T3 represents a period where V(i) reaches a true maximum value Vmax and then decreases, and cVmax(k) remains constant at Vmax and, therefore, AUCₚᵣₑ(k) is also constant, but in the post-peak region, AUCₚₒₛₜ(k) increases with k. T4 represents a period after V(i) has passed the baseline end point (k₂), and both AUCₚᵣₑ(k) and AUCₚₒₛₜ(k) are constant. Figure 3B illustrates changes over time in AUCₚᵣₑ(k) and AUCₚₒₛₜ(k) together with V(i).

**[Table 1]**

| Section | T1 | T2 | T3 | T4 |
|---|---|---|---|---|
| cVmax(k) | - | V(k) | Vmax | Vmax |
| AUCₚᵣₑ(k) | Zero | Rise | Constant value | Constant value |
| AUCₚₒₛₜ(k) | Zero | Zero | Rise | Constant value |

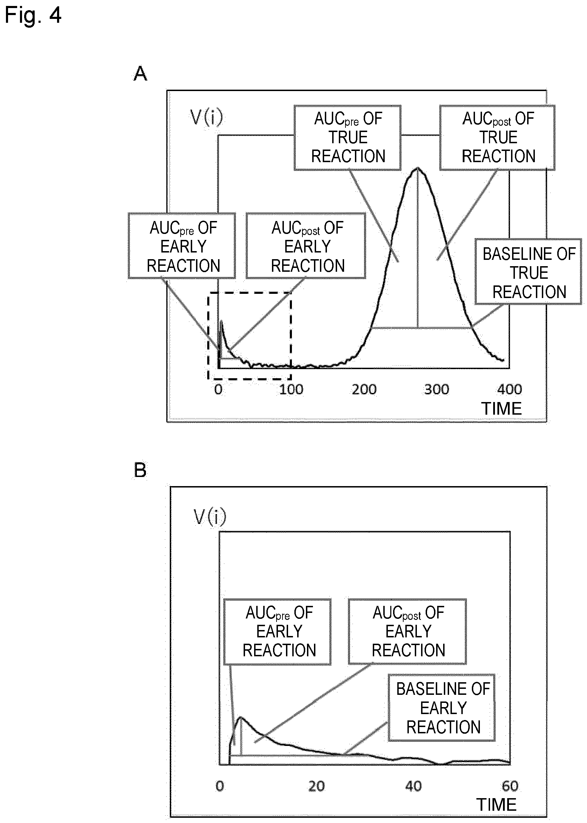

Figure 4 illustrates a pre-peak AUC and a post-peak AUC of V(i) of an early reaction and a true reaction in a coagulation reaction having the early reaction. Figure 4A shows V(i) of an entire coagulation reaction including the early reaction and the true reaction, and Figure 4B is an enlarged view of V(i) of the early reaction. V(i) increases rapidly immediately after the reaction to form a small peak of the early reaction, and then gradually decreases to approach zero. The pre-peak AUC (AUCₚᵣₑ) and the post-peak AUC (AUCₚₒₛₜ) of the early reaction are smaller than those of a subsequent true reaction.

In this manner, AUCₚᵣₑ(k) and AUCₚₒₛₜ(k) change in accordance with the progress of the coagulation reaction. Therefore, a state of progress of the coagulation reaction can be comprehended based on AUCₚᵣₑ(k) and AUCₚₒₛₜ(k).

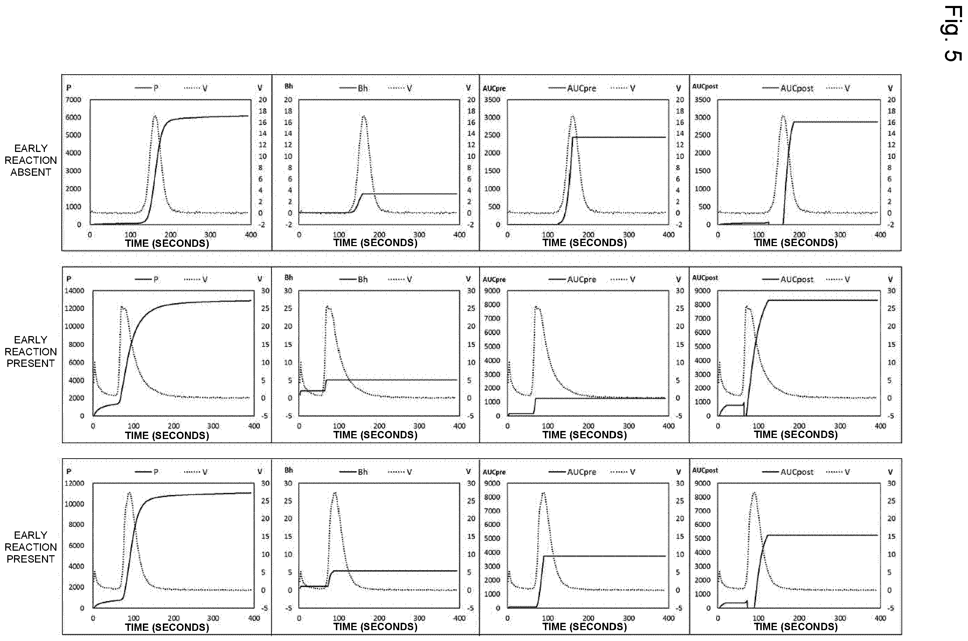

Furthermore, based on AUCₚᵣₑ(k) and AUCₚₒₛₜ(k), initial noise such as an early reaction and a true reaction in the coagulation reaction can be distinguished from each other. Figure 5 shows an example of a coagulation reaction having an early reaction and coagulation reactions not having an early reaction. Each drawing in each row shows, from left to right, a change with time of the reaction P, the baseline Bh indicating cVmax(k) × Hr%, AUCₚᵣₑ, and AUCₚₒₛₜ. A first derivative V is shown together in each drawing. When the coagulation reaction does not have an early reaction (top row), Bh and AUCₚᵣₑincrease as V increases, and become constant once V reaches a peak top (Vmax). On the other hand, AUCₚₒₛₜ increases after V reaches Vmax and becomes constant once V equals or falls below Bh. When the coagulation reaction has an early reaction (middle row and bottom row), Bh increases in two stages being the early reaction and a true reaction. AUCₚᵣₑand AUCₚₒₛₜ rise once during the early reaction, rise again in response to a shift of Bh due to an incidence of the true reaction, and then become constant.

In this manner, AUCₚᵣₑ(k) may become constant once V(i) reaches the peak top and, subsequently, AUCₚₒₛₜ(k) may also become constant in response to both an initial noise such as an early reaction and a true reaction. However, since the peak of V(i) which appears with respect to the initial noise has a relatively small height and width, both AUCₚᵣₑ(k) and AUCₚₒₛₜ(k) are limited to relatively small values. In addition, since AUCₚᵣₑ(k) and AUCₚₒₛₜ(k) with respect to the peak of the initial noise are reset by the incidence of the true reaction, AUCₚᵣₑ(k) and AUCₚₒₛₜ(k) are not kept at constant values for a long time.

Therefore, the true reaction can be detected from V(i) based on the values of the pre-peak AUC and the post-peak AUC. In the method according to the present invention, when the pre-peak AUC and the post-peak AUC exceed a predetermined threshold and the state continues for a certain period or longer, the peak used to calculate the pre-peak AUC and the post-peak AUC is considered a peak of the true reaction.

More specifically, in step 3) of the method according to the present invention, cVmax(k) is detected as a true maximum value Vmax of V(i) when the pre-peak AUC and the post-peak AUC of V(i) calculated in step 2) are both equal to or higher than a first threshold AUCₜₕ₁ and a period L during which the respective AUCs have continued to be a constant value reaches a predetermined length. Vmax denotes the maximum value of the true reaction at V(i).

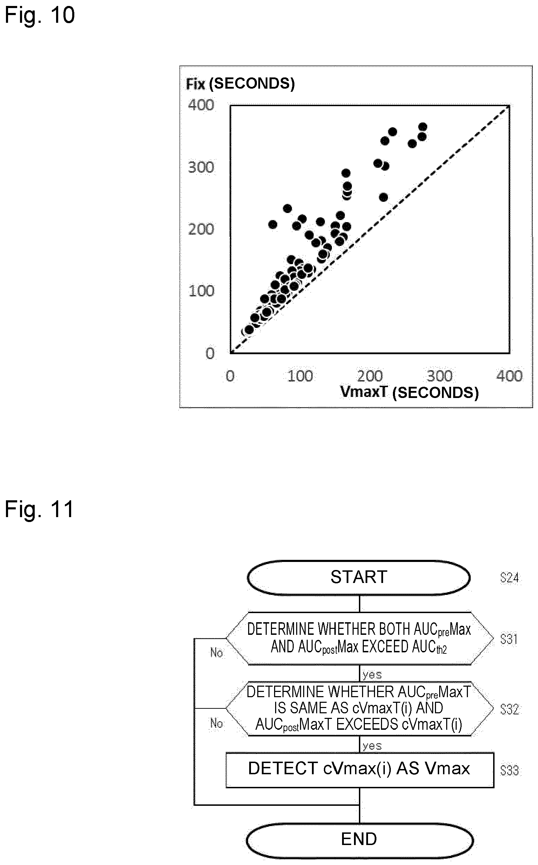

While the first threshold AUCₜₕ₁ may be set to different values for the pre-peak AUC and the post-peak AUC, setting a common threshold for both AUCs may suffice. AUCₜₕ₁ can be appropriately set in consideration of a magnitude of a coagulation reaction or a magnitude of noise of a subject specimen. Therefore, AUCₜₕ₁ can be appropriately set in accordance with the types of a reagent and a device used for coagulation reaction measurement. Under standard APTT measurement conditions, AUCₜₕ₁ is preferably in the range of approximately 1.5 to 2.5 times the maximum value of the pre-peak AUC of the early reaction or, for example, from 250 to 420. If AUCₜₕ₁ is set smaller, although Vmax can be detected even from a specimen having a small coagulation reaction, a risk of erroneous detection of an early reaction increases. In such a case, providing the detection exclusion range described earlier enables an erroneous detection of the early reaction to be prevented. On the other hand, if AUCₜₕ₁ is set to a larger value, although an erroneous detection of the early reaction can be prevented, it becomes difficult to detect Vmax with a specimen having a small coagulation reaction. In such a case, Vmax of a specimen having a small coagulation reaction can be detected by performing a procedure such as that shown in Figure 11 to be described later.

L represents a period during which the pre-peak AUC and the post-peak AUC have respectively remained constant. For example, if an initial value of L is 0 and both the pre-peak AUC and the post-peak AUC at a measurement point or time k are the same as previously calculated values, L increases to L = L + 1, but if one of or both of the pre-peak AUC and the post-peak AUC differ (increase or decrease) from the previously calculated values, L is reset to L = 0.

Preferably, in step 3), L = L + 1 is adopted if AUCₚᵣₑ(k) and AUCₚₒₛₜ(k) are both AUCₜₕ₁ or higher, and if AUCₚᵣₑ(k) = AUCₚᵣₑ(k - x) and AUCₚₒₛₜ(k) = AUCₚₒₛₜ(k - x) are both satisfied, but otherwise, L = 0 is adopted. When L reaches a predetermined value Lₜₕ, cVmax(k) is detected as the true maximum value Vmax of V(i). In this case, the latest measurement point or time with respect to V(i) is k, and the previous measurement point or time is k - x, where x > 0. x can be appropriately set in accordance with a time frame for comparing with a previously calculated value. For example, when k denotes the number of measurement points, x = 1 may be set to compare latest AUCₚᵣₑ(k) with AUCₚᵣₑ(k - 1) having been calculated at an immediately previous measurement point, or x may be set to 2, 3, or more in order to facilitate the calculation process. The same applies when k denotes time. While a value of Lₜₕ can be set as appropriate, Lₜₕ preferably ranges from 0.5 to 2 seconds under standard APTT measurement conditions.

In order to eliminate an erroneous detection of initial noise such as an early reaction, Vmax detected in a premature measurement range or time domain (for example, the detection exclusion range described earlier) where a true reaction cannot possibly occur can be excluded as an error. In this case, if the time with respect to the detected Vmax (in other words, cVmaxT(k)) is included in the detection exclusion range, this means that Vmax was not detected. When setting the detection exclusion range, since AUCₜₕ₁ can be set relatively small, the true maximum value Vmax can even be detected from a specimen having a small coagulation reaction.

The detection of the true maximum value Vmax means that a reaction measured at the measurement point or the time k is not an initial noise such as an early reaction but a true coagulation reaction. Therefore, the peak of V(i) having the detected Vmax as a peak top is considered to be V(i) of the true reaction. The time when Vmax is detected is at a point where AUCₚₒₛₜ(k) becomes a constant value or, in other words, after V(i) drops to or below the baseline and, therefore, the coagulation reaction has reached an end stage at this point. Therefore, in the method according to the present invention, after the time when Vmax is detected, the coagulation reaction measurement of the subject specimen can be ended after acquiring measurement data necessary for calculating a coagulation time. For example, the coagulation reaction measurement of the subject specimen can be ended at a time point where Vmax is detected. Further, for example, the coagulation reaction measurement of the subject specimen can be ended after Vmax is detected at a time point where V(i) has sufficiently dropped. Using the coagulation reaction measured by the end of the measurement in the form of, for example, P(i) or V(i) enables the coagulation time of the subject specimen to be accurately calculated.

On the other hand, in the method according to the present invention, when the true maximum value Vmax is not detected at the measurement point or the time k in step 3) or, in other words, when either the pre-peak AUC or the post-peak AUC does not reach AUCₜₕ₁ at k or when L does not reach a predetermined length, the coagulation reaction measurement is continued and steps 1) to 3) are repeated once again. Preferably, steps 1) to 3) are repeated assuming that k = k + x (x is as described above). This procedure is repeated as long as Vmax is detected or as long as k does not exceed a measurement end point or time (a maximum measurement point or time at which a coagulation reaction measurement of a subject specimen defined in advance is performed). When Vmax is detected or k exceeds the measurement end point or time, the iterative process of steps 1) to 3) ends. The measurement end point or time can be set as appropriate and preferably ranges from 4 to 8 minutes under standard APTT measurement conditions.

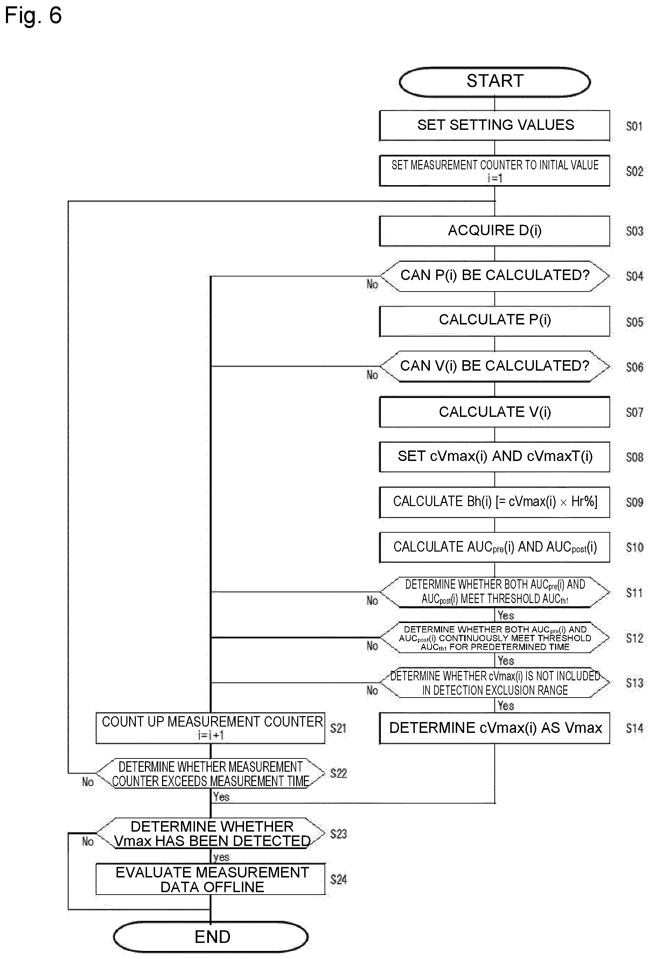

As an embodiment of the present invention, Figure 6 shows a flow chart illustrating a procedure for detecting a true coagulation reaction in accordance with the method of the present invention.
S01: Set setting values (setting values: Hr, AUCₜₕ₁, Lₜₕ, and the like)
S02: Set measurement counter to "i = 1" (initial value)
S03: Acquire measurement data D(i)
S04: Proceed to S05 when reaction P(i) can be calculated (since predetermined number of D(i) is required for smoothing)
S05: Calculate P(i)
S06: Proceed to S07 when first derivative V(i) can be calculated (since predetermined number of P(i) is required for differentiation)
S07: Calculate V(i)
S08: Set cVmax (i) (set i which produces cVmax(i) as cVmaxT(i))
S09: Calculate baseline Bh(i) (= cVmax(i) × Hr%) with respect to cVmax(i)
S10: Calculate AUCₚᵣₑ(i) and AUCₚₒₛₜ(i)
S11: Determine whether both AUCₚᵣₑ(i) and AUCₚₒₛₜ(i) meet threshold AUCₜₕ₁
S12: Determine whether both AUCₚᵣₑ(i) and AUCₚₒₛₜ(i) meet threshold AUCₜₕ₁ and maintain a constant value (whether L ≥ Lₜₕ is satisfied)
S13: This step is not essential: Determine whether or not cVmaxT(i) is in detection exclusion range
S14: If cVmaxT(i) is not included in detection exclusion range, determine cVmax(i) as true maximum value Vmax (maximum value of true reaction) of V(i)
S21: Count up measurement counter
S22: Determine whether measurement counter exceeds set measurement time and, if not, return to S03
S23: If measurement counter exceeds measurement time, end measurement and determine presence or absence of detection of Vmax
S24: Evaluate measurement data offline based on obtained calculated value

When a coagulation ability of a subject specimen is low, the reaction may not be completed and P(i) may continue to rise even at the measurement end point or time. In such a case, since either a peak top of V(i) of a true reaction does not appear or V(i) does not drop sufficiently after the peak top, either the pre-peak AUC does not reach a certain value and the post-peak AUC is 0 or the post-peak AUC does not reach a constant value even though the pre-peak AUC reaches a constant value and, as a result, the method according to the present invention may fail to detect Vmax by the measurement end point or time. In consideration thereof, in an embodiment of the method according to the present invention, when k exceeds the measurement end point or time without detecting the true maximum value Vmax, cVmax(k) is detected as the true maximum value Vmax of V(i) if AUCₚᵣₑ(k) at the measurement end point is equal to or higher than AUCₜₕ₁. By the present procedure, it is possible to detect a true reaction of a subject specimen of which a reaction does not end within the measurement end point or time. When necessary, by outputting information indicating that Vmax had been detected in the present procedure together with Vmax, additional information related to a coagulation ability of the subject specimen can be provided.

When a coagulation reaction of a subject specimen is relatively small, Vmax may not be detected by the measurement end point or time because the pre-peak AUC or the post-peak AUC does not reach AUCₜₕ₁. In consideration thereof, in an embodiment of the method according to the present invention, when k exceeds the measurement end point or time without detecting the true maximum value Vmax, and when maximum values of the pre-peak AUC and the post-peak AUC equal or exceed a second threshold AUCₜₕ₂, a measurement point or time at which the pre-peak AUC is maximized is the same as cVmaxT(k), and a measurement point or time at which the post-peak AUC is maximized is later than cVmaxT(k), then cVmax(k) is detected as the true maximum value Vmax of V(i) (for example, a procedure shown in Figure 11 to be described later is used). AUCₜₕ₂ is preferably smaller than AUCₜₕ₁ and preferably ranges from 10% to 30% of AUCₜₕ₁. By the present procedure, a true reaction of a subject specimen having a relatively low blood coagulation ability can be detected. When necessary, by outputting information indicating that Vmax had been detected in the present procedure together with Vmax, additional information related to a coagulation ability of the subject specimen can be provided.

When a coagulation reaction of a subject specimen is absent or extremely small, or for other reasons, a true reaction of the subject specimen may be unclear. In such a case, it is difficult to distinguish between the true reaction and noise. A specimen with an unclear true reaction can typically be recognized by the fact that the pre-peak AUC does not reach AUCₜₕ₁ (and even AUCₜₕ₂). Alternatively, such a specimen can be recognized by the fact that V(i) does not exceed the threshold Vₜₕ described earlier. For example, by starting step 2) only when V(i) has reached Vₜₕ, noise can be prevented from being erroneously detected. In a specimen in which a true reaction is unclear, the true reaction is excluded along with noise and Vmax may not be detected. In that case, by outputting a result that Vmax is not detected and information indicating that Vmax is not detected because either a pre-peak AUC exceeding the threshold AUCₜₕ₁ cannot be detected or V(i) equal to or higher than Vₜₕ cannot be detected, additional information related to a coagulation ability of the subject specimen can be provided.

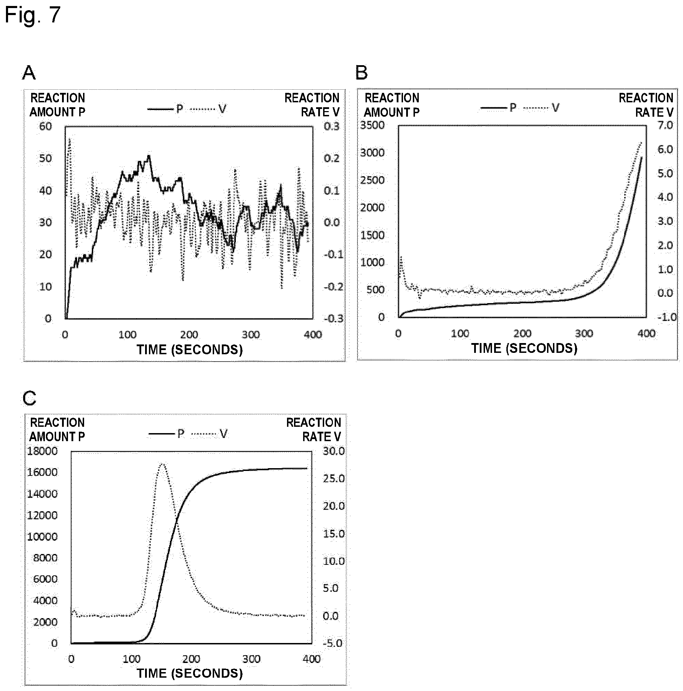

As an example, Figures 7A to 7C show a reaction P and a first derivative V of three subject specimens having different coagulation abilities. Figure 7A shows a specimen in which a true reaction is unclear, Figure 7B shows a specimen in which a true reaction did not end within the measurement end point or time, and Figure 7C shows a specimen in which a true reaction was detected. These specimens can be classified based on AUCₚᵣₑ(k) and AUCₚₒₛₜ(k) as follows.
Figure 7A: AUCₚᵣₑ(k) (and AUCₚₒₛₜ(k)) reaches neither AUCₜₕ₁ nor AUCₜₕ₂ → No reaction
Figure 7B: AUCₚᵣₑ(k) (or AUCₚₒₛₜ(k)) reaches AUCₜₕ₁ but does not reach a constant value → Reaction in progress
Figure 7C: AUCₚᵣₑ(k) and AUCₚₒₛₜ(k) reach AUCₜₕ₁ and are continuously constant values → Reaction detected

### [2. Calculation of blood coagulation time and other analysis]

In an embodiment, in the method according to the present invention, after detecting a true reaction of a subject specimen, a coagulation time of the subject specimen can be further calculated using the detected true reaction. The true coagulation reaction acquired in the method according to the present invention, such as P(i) or V(i), can be used to calculate a blood coagulation time or used in other various blood analyses.

In an example, in the method according to the present invention, a true maximum value Vmax can be detected using V(i) acquired from data of coagulation reaction measurement, and a coagulation time can be further calculated using V(i). In another example, in the method according to the present invention, P(i) and V(i) can be acquired from the data of coagulation reaction measurement, the true maximum value Vmax can be detected using V(i), and then a coagulation time can be calculated using P(i) and/or V(i).

In an embodiment, in the method according to the present invention, coagulation reaction measurement of a subject specimen can be ended at a time point where Vmax is detected and a coagulation time can be calculated using P(i) and/or V(i) of a true reaction acquired up to that point. In an embodiment, a coagulation time can be calculated using P(i) and/or V(i) after a rising edge of a peak containing the detected Vmax such as P(i) and/or V(i) at i ≥ k₁.

In another embodiment, in the method according to the present invention, coagulation reaction measurement of a subject specimen can be continued even after Vmax is detected, and data related to a coagulation reaction necessary for the calculation of a coagulation time or other analyses can be acquired.

The method for calculating a coagulation time from a coagulation reaction acquired by the method according to the present invention is not particularly limited. Examples of the method for calculating the coagulation time include: a method for calculating a time point where P(i) reaches N% of a maximum value Pmax as the coagulation time (so-called percentage detection method); a method for calculating a time point where V(i) reaches a maximum value Vmax or N% thereof as the coagulation time (so-called differential method); a method for calculating the coagulation time based on a change over time of an integrated value of P(i) in a small time slot (refer to Patent Literature 3 and Japanese Patent Application No. 2019-237427); a method for calculating the coagulation time based on a weighted average time of V(i) (refer to Japanese Patent Application No. 2020-039344); and a method for calculating, with an origin of calculation Te being a time point where V(i) reaches a predetermined value after reaching the maximum value Vmax, a time point where P(i) reaches N% of P(Te) as the coagulation time (refer to Japanese Patent Application No. 2020-068877).

### [3. Application to other coagulation reaction measurement methods]

A method for detecting a blood coagulation reaction according to the present invention has been described above using a coagulation reaction measurement based on a scattered light quantity as an example. However, a person skilled in the art should be able to apply the method according to the present invention to methods for detecting a blood coagulation reaction using other coagulation reaction measurement methods (for example, blood coagulation reaction measurement methods based on transmittance, absorbance, viscosity, or the like) and, therefore, such an application is included in a scope of the present invention. For example, a reaction P(i) obtained from a coagulation reaction curve with an inverse sigmoidal shape based on a scattered light quantity has reverse polarity with respect to a coagulation reaction curve based on the scattered light quantity described above. In such a case, it should be obvious to those skilled in the art that, in the steps 1) to 3) described above, the signs of P(i) and V(i) are to be reversed, a minimum value cVmin(k) of V(i) up to k is to be calculated instead of cVmax(k), an area on the curve (AOC) is to be calculated instead of the area under the curve (AUC), and the like.

### [4. Programs and apparatus]

The method for detecting a blood coagulation reaction according to the present invention described above can be automatically performed using a computer program. Therefore, an aspect of the present invention is a program for performing the method for detecting a blood coagulation reaction according to the present invention described above. In addition, the series of steps of the method according to the present invention described above can be automatically performed by an automated analyzer. Therefore, an aspect of the present invention is an apparatus for performing the method for detecting a blood coagulation reaction according to the present invention described above.

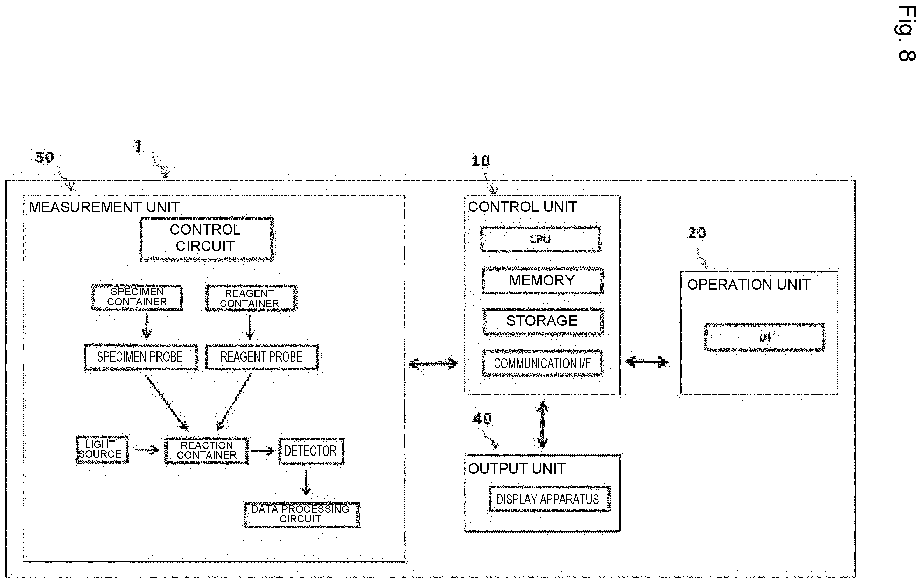

An embodiment of the apparatus according to the present invention will be described below. The embodiment of the apparatus according to the present invention is an automated analyzer 1 such as that shown in Figure 8. The automated analyzer 1 includes a control unit 10, an operation unit 20, a measurement unit 30, and an output unit 40.

The control unit 10 controls operations of the automated analyzer 1 as a whole. The control unit 10 can be constituted of, for example, a personal computer (PC). The control unit 10 includes a CPU, a memory, a storage, a communication interface (I/F), and the like and performs processing of a command from the operation unit 20, control of operations of the measurement unit 30, storage and data analysis of measured data received from the measurement unit 30, storage of an analysis result, control of output of measured data and/or an analysis result by the output unit 40, and the like. Furthermore, the control unit 10 may be connected to other devices such as an external medium and a host computer. In the control unit 10, a PC which controls operations of the measurement unit 30 may be or may not be the same as a PC which performs analysis of measured data.

The operation unit 20 acquires input from an operator and transmits obtained input information to the control unit 10. For example, the operation unit 20 includes a user interface (UI) such as a keyboard or a touch panel. Under the control of the control unit 10, the output unit 40 outputs measurement data of the measurement unit 30, analysis results thereof including detection results of P(i), V(i), and a true coagulation reaction (Vmax and the like), a coagulation time of a blood specimen, and the like. For example, the output unit 40 includes a display apparatus such as a display.

The measurement unit 30 executes a series of operations for a blood coagulation test and acquires measured data of a coagulation reaction of a sample including a blood specimen. The measurement unit 30 includes various equipment and analysis modules necessary for a blood coagulation test such as a specimen container for storing a blood specimen, a reagent container for storing a test reagent, a reaction container for a reaction between the specimen and the reagent, a probe for dispensing the blood specimen and the reagent to the reaction container, a light source, a detector for detecting scattered light or transmitted light from the sample in the reaction container, a data processing circuit which sends data from the detector to the control unit 10, and a control circuit which receives an instruction of the control unit 10 and which controls operations of the measurement unit 30.

The control unit 10 performs an analysis of a coagulation reaction of a specimen based on data measured by the measurement unit 30. The present analysis can include acquisition of P(i) and V(i), detection of a true coagulation reaction (Vmax and the like), and calculation of a coagulation time using the detected true coagulation reaction described above. Alternatively, the coagulation reaction curve P(i) or the first derivative V may be created by the control unit 10 based on measured data from the measurement unit 30 or may be created by another device such as the measurement unit 30 and sent to the control unit 10. The control unit 10 may store setting values such as parameters used in detection of a true coagulation reaction, for example, AUCₜₕ₁ and Lₜₕ or the control unit 10 may import, when performing an analysis, the setting values stored in an external device or on a network.

The present analysis described above can be implemented by a program for performing the method according to the present invention. Therefore, the control unit 10 can include a program for performing the method for detecting a blood coagulation reaction according to the present invention.

An analysis result obtained by the control unit 10 is sent to the output unit 40 to be output. The output can take any form such as a display on a screen, a transmission to a host computer, a printout, or the like. Output information from the output unit can include P(i), V(i), a detection result of a true coagulation reaction, and a coagulation time. Types of output information from the output unit can be controlled by the program according to the present invention.

In an embodiment of the apparatus according to the present invention, the measurement unit 30 continues measurement of a subject specimen until an end of a coagulation reaction and data is sequentially sent to the control unit 10. The control unit 10 sequentially performs calculations for acquiring P(i) and V(i) and calculations of cVmax(k), cVmaxT(k), AUCₚᵣₑ(k), and AUCₚₒₛₜ(k), and determines whether or not L ≥ Lₜₕ is satisfied and detects Vmax. When Vmax is detected, the control unit 10 further calculates a coagulation time of a subject specimen. An obtained analysis result is sent to the output unit to be output. For example, P(i) and V(i) are sequentially output in parallel with measurements and, when necessary, cVmax(k), cVmaxT(k), AUCₚᵣₑ(k), and AUCₚₒₛₜ(k) are also sequentially output. After Vmax is detected, Vmax, a time thereof, and a coagulation time are output when appropriate.

### Examples

While the present invention will be described below in greater detail by citing Examples, it is to be understood that the present invention is not limited by the following Examples.

### Example 1 Parameters reflecting coagulation reaction

### 1) Subject specimen

The following 187 examples were used as subject specimens: normal plasma (10 examples), heparin-dosed plasma (47 examples), LA-positive plasma (11 examples), coagulation factor-deficient plasma (14 examples), inhibitor plasma (41 examples), low-concentration fibrinogen plasma (5 examples), and other prolonged-coagulation time plasma (59 examples). An early reaction was observed in six of these examples.

### 2) Coagulation reaction measurement

Coagpia APTT-N (manufactured by SEKISUI MEDICAL CO., LTD.) which is a reagent for APTT measurement was used as a reagent for measurement and Coagpia APTT-N Calcium Chloride Solution (manufactured by SEKISUI MEDICAL CO., LTD.) was used as a calcium chloride solution. A coagulation reaction measurement of samples including a specimen was performed using an Automated Coagulation Analyzer CP3000 (manufactured by SEKISUI MEDICAL CO., LTD.). After heating 50 µL of a specimen in a cuvette at 37°C for 45 seconds, 50 µL of the reagent for measurement at approximately 37°C was added and, after a lapse of 171 seconds, 50 µL of a 25 mM calcium chloride solution was added to start a coagulation reaction. The reaction was performed at 37°C. In the measurement of the coagulation reaction, the cuvette was irradiated with light with a wavelength of 660 nm from an LED light source, and a scattered light quantity of light scattered 90 degrees to the side was measured at 0.1-second intervals. A maximum measurement time was set to 400 seconds (measurement point i = 1 to 4,000).

### 3) Acquisition of reaction P(i) and reaction rate V(i)

After performing smoothing including denoising with respect to photometry data from each specimen, zero point adjustment was performed so that a scattered light quantity at a time point of start of photometry equaled zero to create a reaction P(i). A first derivative V(i) was calculated from P(i).

### 4) APTT calculation

A coagulation time (APTT) of the subject specimen was calculated by a percentage method. A time at which P(i) reached 50% of a maximum value (Pmax) thereof was adopted as the coagulation time.

### Example 1 Parameters reflecting coagulation reaction

### 1) Calculation of parameters

Using obtained P(i) and V(i) (i = 21 to 3,980), the following parameters were calculated by setting a height ratio (Hr) to 20%.
Vmax: maximum value of V(i)
VmaxT: time of Vmax
Pmax: maximum value of P(i)
AUCₚᵣₑ: pre-peak AUC of V(i)
AUCₚₒₛₜ: post-peak AUC of V(i)

Table 2 shows an example of calculated parameters. The nine specimens shown in Table 2 were as follows.
Specimen 1: specimen with smallest Pmax
Specimen 2: specimen with smallest VmaxT
Specimen 3: specimen with bimodal V(i)
Specimens 4 to 9: specimens in which early reaction had appeared

**[Table 2]**

| **Content** | **VmaxT** | **Pmax** | **Vmax** | **AUCₚᵣₑ** | **AUCₚₒₛₜ** |
|---|---|---|---|---|---|
| Specimen 1 | 139.0 | 569 | 1.7 | 207 | 285 |
| Specimen 2 | 21.0 | 4,133 | 35.3 | 799 | 2,669 |
| Specimen 3 | 60.9 | 7,046 | 6.5 | 377 | 6,076 |
| Specimen 4 | 165.3 | 13,435 | 10.2 | 4,044 | 8,163 |
| Specimen 5 | 70.3 | 12,885 | 25.7 | 1,265 | 8,322 |
| Specimen 6 | 90.3 | 11,071 | 27.4 | 3,748 | 5,232 |
| Specimen 7 | 259.9 | 12,210 | 11.9 | 4,518 | 6,097 |
| Specimen 8 | 274.6 | 12,280 | 12.0 | 5,144 | 5,644 |
| Specimen 9 | 166.2 | 9,837 | 19.8 | 3,700 | 4,717 |

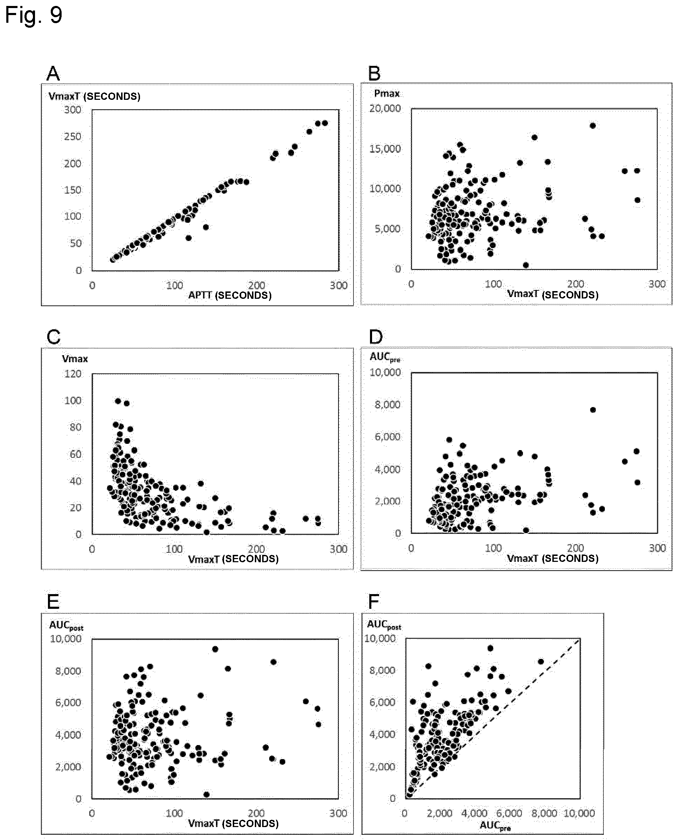

Figures 9A to 9F show relationships among parameters. APTTs of the 187 subject specimens were distributed between a minimum value 24.9 seconds and a maximum value 283.7 seconds, and VmaxT had an approximately linear relationship with APTT (Figure 9A). In Figure 9A, the two specimens having deviated from the linear relationship were coagulation factor VIII (FVIII)-deficient specimens having bimodal V(i) with a larger first peak. There was no clear trend between the maximum value Pmax of P(i) and VmaxT (Figure 9B). The relationship between Vmax and VmaxT tended to be inversely proportional (Figure 9C). This tendency indicates that the peak of V(i) becomes large when the progress of a coagulation reaction is fast and becomes small when the progress of the coagulation reaction is slow. There was no clear relationship among pre-peak AUC, post-peak AUC, and VmaxT (Figures 9D and 9E). The post-peak AUC tended to be larger than the pre-peak AUC (Figure 9F).

### 2) Parameters of subject specimens having early reaction

With respect to the subject specimens (specimens 4 to 9 in Table 2) having an early reaction, after determining an early reaction part and a true reaction part of the acquired coagulation reaction curve, the parameters Vmax, VmaxT, Pmax, pre-peak AUC (AUCₚᵣₑ), and post-peak AUC (AUCₚₒₛₜ) were calculated. Table 3 shows results of the calculation. It was confirmed that VmaxT of the true reaction part ranged from about 70 seconds to 275 seconds, but the VmaxT of the early reaction part was always about 4 seconds and appeared in an early stage of the measurement.

**[Table 3]**

| **Content** | **Specimen number** | **VmaxT** | **Pmax** | **Vmax** | **AUCₚᵣₑ** | **AUCₚₒₛₜ** |
|---|---|---|---|---|---|---|
| Early reaction part | Specimen 4 | 4.1 | 186 | 2.6 | 44 | 138 |
| | Specimen 5 | 4.1 | 942 | 10.0 | 168 | 756 |
| | Specimen 6 | 4.1 | 488 | 5.5 | 92 | 386 |
| | Specimen 7 | 4.3 | 396 | 3.3 | 59 | 332 |
| | Specimen 8 | 4.4 | 364 | 2.9 | 53 | 307 |
| | Specimen 9 | 4.2 | 255 | 2.6 | 44 | 207 |
| True reaction part | Specimen 4 | 165.3 | 13,435 | 10.2 | 4,044 | 8,163 |
| | Specimen 5 | 70.3 | 12,885 | 25.7 | 1,265 | 8,322 |
| | Specimen 6 | 90.3 | 11,071 | 27.4 | 3,748 | 5,232 |
| | Specimen 7 | 259.9 | 12,210 | 11.9 | 4,518 | 6,097 |
| | Specimen 8 | 274.6 | 12,280 | 12.0 | 5,144 | 5,644 |
| | Specimen 9 | 166.2 | 9,837 | 19.8 | 3,700 | 4,717 |

### Example 2 Real-time detection of true coagulation reaction-1

Using the coagulation reaction data of the 187 specimens measured in Example 1, real-time detection of Vmax was performed in accordance with the procedure shown by the flow chart in Figure 6.

### 1) Determination of threshold AUCₜₕ₁

As a result of Example 1, minimum values of AUCₚᵣₑ and AUCₚₒₛₜ among the subject specimens were 207 and 285 in specimen 1 with smallest Pmax. On the other hand, a maximum value of AUCₚᵣₑ in the early reaction part of the specimens 4 to 9 with an early reaction was 168 in specimen 5. Excessively low AUCₜₕ₁ causes the early reaction part in specimen 5 and the other specimens to be erroneously detected, but excessively large AUCₜₕ₁ prevents small reactions in specimen 1, specimen 3, and the like from being detected. From the results described above, AUCₜₕ₁ was determined as 255 or, in other words, around 1.5 times the maximum value of the early reaction part (168 in specimen 5).

### 2) Detection condition

A real-time detection of Vmax was performed under the following conditions.
i = 21 to Vmax detection (maximum 3,980)
Hr(%): 20%
AUCₜₕ₁: 255
Lₜₕ: 10 (1 second)
Detection exclusion range (step S13): Absent

### 3) Result

Vmax was detected from a true reaction in 186 specimens with the exception of specimen 1.

### Example 3 Real-time detection of true coagulation reaction-2 (low threshold, detection exclusion range present)

From the results of Example 1, it was confirmed that the peak of the early reaction appeared in an early stage of measurement. In consideration thereof, a real-time detection of Vmax was performed under conditions that, although a reaction smaller than specimen 1 of Example 1 can be detected, detection of a reaction in a section where an appearance of an early reaction is expected is not performed (low threshold, step S13 present). Specifically, a real-time detection of Vmax was performed under the same conditions as Example 2, with the exception of changes made as follows.
AUCₜₕ₁: 100
Detection exclusion range (step S13): Present. Vmax detected only when cVmaxT(i) is 50 (5 seconds) or later

Vmax was detected from a true reaction in all 187 specimens. Figure 10 shows a plot of time (Fix) at which Vmax was detected in the present Example with respect to VmaxT measured in Example 1. Since Fix in all specimens was a later time than VmaxT, it was confirmed that the true reaction could be detected correctly by the procedure of the present Example. Furthermore, from the results of the present Example, it was shown that by setting the detection exclusion range (step S13), the true reaction can be correctly detected even in a specimen having a small reaction.

### Example 4 Real-time detection of true coagulation reaction-3 (high threshold, offline detection present)

Assuming a case where an early reaction larger than that of specimens 4 to 9 of Example 1 appears, AUCₜₕ₁ was set higher than in 1) in Example 2. It was predicted that, by setting AUCₜₕ₁ high, a true reaction will no longer be detected in a part of the specimens (for example, specimen 1) by the procedure shown in Figure 6. Therefore, the procedure shown in Figure 11 was incorporated between S24 and the end in Figure 6. Specifically, a real-time detection of Vmax was performed under the same conditions as Example 2, with the exception of changes made as follows.
AUCₜₕ₁: 510
AUCₜₕ₂: 100

The following procedures are performed in S24:
S31: Determine whether maximum values AUCₚᵣₑMax and AUCₚₒₛₜMax of AUCₚᵣₑ(i) and AUCₚₒₛₜ(i) both exceed threshold AUCₜₕ₂
S32: When S31 is satisfied, determine whether a time (AUCₚᵣₑMaxT) where AUCₚᵣₑMax is reached is a same time as cVmaxT (i) and a time (AUCₚₒₛₜMaxT) where AUCₚₒₛₜMax is reached is later than cVmaxT(i)
S33: When S32 is satisfied, detect cVmax(i) as Vmax

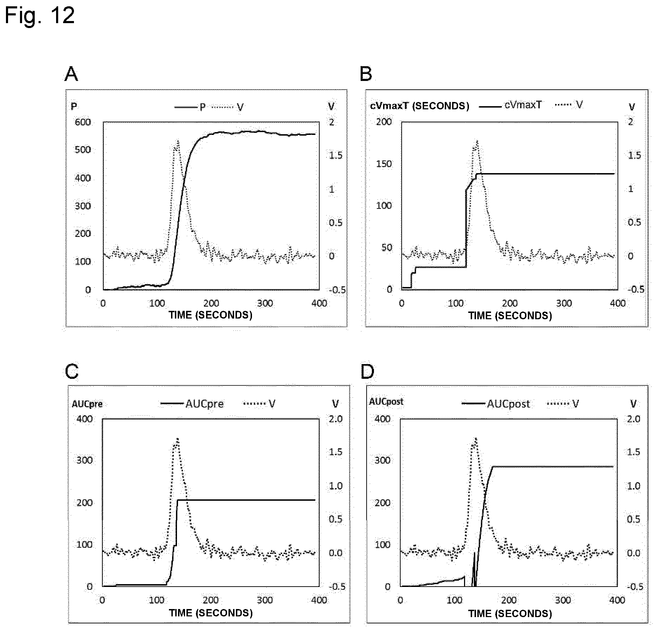

Vmax was detected via S24 in 12 specimens including specimen 1 having smallest Pmax, but Vmax was detected in all the other specimens without involving S24. Figure 12 shows a change over time of the parameters calculated when the procedure of the present Example was performed on specimen 1. As shown in Figures 12B to 12D, cVmaxT(i) was maximized at the time VmaxT of the true maximum value, AUCₚᵣₑ(i) was maximized at VmaxT, and AUCₚₒₛₜ(i) was maximized after VmaxT. It was confirmed that a maximum value of a true reaction was correctly detected at Vmax in 12 specimens including specimen 1 in which Vmax was detected via S24.

## Claims

1. A method for detecting a blood coagulation reaction, comprising:
1) measuring a blood coagulation reaction of a subject blood specimen and acquiring a first derivative V(i) of a coagulation reaction up to a latest measurement point, where i represents a measurement point or time and satisfies i = k₀ to k, k represents a latest measurement point or time with respect to a latest V(i), and k₀ represents any measurement point or time where k₀ ≤ k,
2) calculating areas under the curve (AUCs) before the peak (pre-peak AUC) and after the peak (post-peak AUC) of V(i), wherein a top of the peak of V(i) is cVmax(k) which is a maximum value of V(i) up to i=k; and
3) detecting cVmax(k) as Vmax which is a true maximum value of V(i) when the pre-peak AUC and the post-peak AUC are both equal to or higher than a first threshold AUCₜₕ₁ and a period L during which the pre-peak AUC and the post-peak AUC have continued to be a constant value reaches a predetermined length, wherein this result indicates at least that a true coagulation reaction is correctly detected.

2. The method according to claim 1, wherein
the step 2) comprises calculating cVmax(k), cVmaxT(k), AUCₚᵣₑ(k), and AUCₚₒₛₜ(k), where
cVmax(k) represents a maximum value of V(i) (i = k₀ to k),
cVmaxT(k) represents a measurement point or time where V(i) = cVmax(k) is satisfied,
AUCₚᵣₑ(k) represents a pre-peak AUC at i=k which is an AUC of V(i) from i=k₁ to i=cVmaxT(k),
AUCₚₒₛₜ(k) represents a post-peak AUC at i=k which is an AUC of V(i) from i=cVmaxT(k) to i=k₂,
k₁ represents a latest measurement point or time among measurement points or times where V(i) ≤ cVmax(k) × Hr% is satisfied prior to cVmaxT(k),
k₂ represents an earliest measurement point or time among measurement points or times where V(i) ≤ cVmax(k) × Hr% is satisfied after cVmaxT(k),
0 < Hr < 100, and
the method comprises
repeating the steps 1) to 3), assuming that k = k + x (x > 0) when the Vmax is not detected in the step 3).

3. The method according to claim 2, wherein
the step 3) comprises:
adopting L = L + 1 when both AUCₚᵣₑ(k) and AUCₚₒₛₜ(k) are equal to or larger than AUCₜₕ₁ and, at the same time, when AUCₚᵣₑ(k) = AUCₚᵣₑ(k - x) and AUCₚₒₛₜ(k) = AUCₚₒₛₜ(k - x) where x > 0 are satisfied, but otherwise adopting L = 0; and
detecting cVmax(k) as the Vmax of V(i) when L reaches a predetermined value.

4. The method according to claim 2 or 3, wherein 10 ≤ Hr ≤ 70.

5. The method according to any one of claims 1 to 4, wherein in the step 3), Vmax is not detected when a measurement point or time which equals cVmax(k) is included in a detection exclusion range.

6. The method according to any one of claims 1 to 5, further comprising detecting cVmax(k) as the Vmax of V(i) when the pre-peak AUC at k is equal to or larger than the first threshold AUCₜₕ₁ when k exceeds a measurement end point or time without detecting the Vmax.

7. The method according to any one of claims 1 to 6, further comprising detecting cVmax(k) as the Vmax of V(i) when both the pre-peak AUC and the post-peak AUC at i=k are equal to or larger than a second threshold AUCₜₕ₂, a measurement point or time at which the pre-peak AUC is maximized is the same as a measurement point or time which equals cVmax(k), and a measurement point or time at which the post-peak AUC is maximized is later than the measurement point or time which equals cVmax(k) when k exceeds a measurement end point or time without detecting the Vmax.

8. The method according to any one of claims 1 to 7, further comprising obtaining the coagulation reaction up to the latest measurement point as a reaction P(i).

9. The method according to claim 8, further comprising calculating a blood coagulation time of the subject blood specimen based on P(i) or V(i).

10. The method according to claim 9, wherein i ≥ k₁ is satisfied.

## Patentansprüche

1. Verfahren zur Detektion einer Blutgerinnungsreaktion, umfassend:
1) Messen einer Blutgerinnungsreaktion einer Blutprobe und Ermitteln einer ersten Ableitung V(i) einer Gerinnungsreaktion bis zu einem letzten Messpunkt, wobei i einen Messpunkt oder Zeitpunkt darstellt und i = k₀ bis k erfüllt, wobei k einen letzten Messpunkt oder Zeitpunkt in Bezug auf ein letztes V(i) darstellt und k₀ einen beliebigen Messpunkt oder Zeitpunkt darstellt, für den k₀ ≤ k gilt,
2) Berechnen von Flächen unter der Kurve (AUCs) vor dem Peak (Pre-Peak-AUC) und nach dem Peak (Post-Peak-AUC) von V(i), wobei die Spitze des Peaks von V(i) cVmax(k) ist, was einem Maximalwert von V(i) bis i=k entspricht; und
3) Detektieren von cVmax(k) als Vmax - dem tatsächlichen Maximalwert von V(i) -, wenn sowohl die Pre-Peak-AUC als auch die Post-Peak-AUC gleich oder größer als ein erster Schwellenwert AUCₜₕ₁ sind und ein Zeitraum L, während dessen die Pre-Peak-AUC und die Post-Peak-AUC einen konstanten Wert beibehalten haben, eine vorbestimmte Dauer erreichen, wobei dieses Ergebnis zumindest anzeigt, dass eine tatsächliche Gerinnungsreaktion korrekt detektiert wurde.

2. Verfahren nach Anspruch 1, wobei
Schritt 2) das Berechnen von cVmax(k), cVmaxT(k), AUCₚᵣₑ (k) und AUCₚₒₛₜ (k) umfasst, wobei
cVmax(k) einen Maximalwert von V(i) (i = k₀ bis k) darstellt,
cVmaxT(k) einen Messpunkt oder Zeitpunkt darstellt, an dem V(i) = cVmax(k) erfüllt ist,
AUCₚᵣₑ(k) eine AUC vor dem Peak bei i=k darstellt, welche eine AUC von V(i) von i=k₁ bis i=cVmaxT(k) ist,
AUCₚₒₛₜ(k) eine AUC nach dem Peak bei i=k darstellt, welche eine AUC von V(i) von i=cVmaxT(k) bis i=k₂ ist,
k₁ den spätesten Messpunkt oder Zeitpunkt unter den Messpunkten oder Zeitpunkten darstellt, an denen V(i) ≤ cVmax(k) × Hr% vor cVmaxT(k) erfüllt ist,
k₂ den frühesten Messpunkt oder Zeitpunkt unter den Messpunkten oder Zeitpunkten darstellt, an denen V(i) ≤ cVmax(k) × Hr% nach cVmaxT(k) erfüllt ist,
0 < Hr < 100, und
das Verfahren umfasst:
Wiederholen der Schritte 1) bis 3) unter der Annahme, dass k = k + x (x > 0),
wenn Vmax in Schritt 3) nicht detektiert wird.

3. Verfahren nach Anspruch 2, wobei
Schritt 3) umfasst:
Setzen von L = L + 1, wenn sowohl AUCₚᵣₑ (k) als auch AUCₚₒₛₜ (k) gleich oder größer als AUCth1 sind und gleichzeitig AUCₚᵣₑ (k) = AUCₚᵣₑ (k - x) sowie AUCₚₒₛₜ (k) = AUCₚₒₛₜ (k - x) mit x > 0 erfüllt sind, andernfalls jedoch Setzen von L = 0; und
Detektieren von cVmax(k) als Vmax von V(i), wenn L einen vorbestimmten Wert erreicht.

4. Verfahren nach Anspruch 2 oder 3, wobei 10 ≤ Hr ≤ 70 gilt.

5. Verfahren nach einem der Ansprüche 1 bis 4, wobei in Schritt 3) Vmax nicht detektiert wird, wenn ein Messpunkt oder Zeitpunkt, der cVmax(k) entspricht, in einem Detektions-Ausschlussbereich liegt.

6. Verfahren nach einem der Ansprüche 1 bis 5, ferner umfassend das Detektieren von cVmax(k) als Vmax von V(i), wenn die AUC vor dem Peak bei k gleich oder größer als ein erster Schwellenwert AUCₜₕ₁ ist, sofern k einen Messendpunkt oder -zeitpunkt überschreitet, ohne dass Vmax detektiert wurde.

7. Verfahren nach einem der Ansprüche 1 bis 6, ferner umfassend das Detektieren von cVmax(k) als Vmax von V(i), wenn sowohl die AUC vor dem Peak als auch die AUC nach dem Peak bei i=k gleich oder größer als ein zweiter Schwellenwert AUCₜₕ₂ sind, ein Messpunkt oder Zeitpunkt, an dem die AUC vor dem Peak maximiert ist, derselbe ist wie ein Messpunkt oder Zeitpunkt, der cVmax(k) entspricht, und ein Messpunkt oder Zeitpunkt, an dem die AUC nach dem Peak maximiert ist, später liegt als der Messpunkt oder Zeitpunkt, der cVmax(k) entspricht, sofern k einen Messendpunkt oder - zeitpunkt überschreitet, ohne dass Vmax detektiert wurde.

8. Verfahren nach einem der Ansprüche 1 bis 7, ferner umfassend das Erfassen der Gerinnungsreaktion bis zum spätesten Messpunkt als Reaktion P(i).

9. Verfahren nach Anspruch 8, ferner umfassend das Berechnen einer Blutgerinnungszeit der betreffenden Blutprobe auf der Grundlage von P(i) oder V(i).

10. Verfahren nach Anspruch 9, wobei i ≥ k₁ erfüllt ist.

## Revendications

1. Procédé de détection d'une réaction de coagulation sanguine, comprenant :
1) la mesure d'une réaction de coagulation sanguine d'un échantillon sanguin de sujet et l'acquisition d'une première dérivée V(i) d'une réaction de coagulation jusqu'à un point de mesure le plus récent, où i représente un point ou temps de mesure et satisfait à i = k₀ à k, k représente un point ou temps de mesure le plus récent par rapport à une V(i) la plus récente, et k₀ représente tout point ou temps de mesure où k₀ ≤ k,
2) le calcul d'aires sous la courbe (AUC) avant le pic (AUC pré-pic) et après le pic (AUC post-pic) de V(i), dans lequel un sommet du pic de V(i) est cVmax(k) qui est une valeur maximale de V(i) jusqu'à i=k ; et
3) la détection de cVmax(k) en tant que Vmax qui est une valeur maximale réelle de V(i) lorsque l'AUC pré-pic et l'AUC post-pic sont toutes deux égales ou supérieures à un premier seuil AUCₜₕ₁ et lorsqu'une période L, pendant laquelle l'AUC pré-pic et l'AUC post-pic ont continué à être une valeur constante, atteint une longueur prédéterminée, dans lequel ce résultat indique au moins qu'une réaction de coagulation réelle est correctement détectée.

2. Procédé selon la revendication 1, dans lequel
l'étape 2) comprend le calcul de cVmax(k), cVmaxT(k), AUC_{pré}(k) et AUCₚₒₛₜ(k), où
cVmax(k) représente une valeur maximale de V(i) (i = k₀ à k),
cVmaxT(k) représente un point ou temps de mesure où V(i) = cVmax(k) est satisfait,
AUC_{pré}(k) représente une AUC pré-pic à i=k qui est une AUC de V(i) de i=k₁ à i=cVmaxT(k),
AUCₚₒₛₜ(k) représente une AUC post-pic à i=k qui est une AUC de V(i) de i=cVmaxT(k) à i=k₂,
k₁ représente un point ou temps de mesure le plus récent parmi les points ou temps de mesure où V(i) ≤ cVmax(k) × Hr% est satisfaite avant cVmaxT(k),
k₂ représente un point ou temps de mesure le plus ancien parmi les points ou temps de mesure où V(i) ≤ cVmax(k) × Hr% est satisfaite après cVmaxT(k),
0 < Hr < 100, et
le procédé comprend la répétition des étapes 1) à 3), en supposant que k = k + x (x > 0) lorsque la Vmax n'est pas détectée à l'étape 3).

3. Procédé selon la revendication 2, dans lequel
l'étape 3) comprend :
le choix de L = L + 1 lorsque AUC_{pré}(k) et AUCₚₒₛₜ(k) sont toutes deux égales ou supérieures à AUCₜₕ₁ et, en même temps, lorsque AUC_{pré}(k) = AUC_{pré}(k - x) et AUCₚₒₛₜ(k) = AUCₚₒₛₜ(k - x) où x > 0 sont satisfaites, sinon le choix de L = 0 ; et
la détection de cVmax(k) en tant que la Vmax de V(i) lorsque L atteint une valeur prédéterminée.

4. Procédé selon la revendication 2 ou 3, dans lequel 10 ≤ Hr ≤ 70.

5. Procédé selon l'une des revendications 1 à 4, dans lequel, à l'étape 3), Vmax n'est pas détectée lorsqu'un point ou temps de mesure qui est égal à cVmax(k) est présent dans une plage d'exclusion de détection.

6. Procédé selon l'une des revendications 1 à 5, comprenant en outre la détection de cVmax(k) en tant que la Vmax de V(i) lorsque l'AUC pré-pic à k est égale ou supérieure au premier seuil AUCₜₕ₁ lorsque k dépasse un point ou temps de fin de mesure sans détecter la Vmax.

7. Procédé selon l'une des revendications 1 à 6, comprenant en outre la détection de cVmax(k) en tant que la Vmax de V(i) lorsque l'AUC pré-pic et l'AUC post-pic à i=k sont toutes deux égales ou supérieures à un second seuil AUCₜₕ₂, un point ou temps de mesure auquel l'AUC pré-pic est maximisée est identique à un point ou temps de mesure qui est égal à cVmax(k), et un point ou temps de mesure auquel l'AUC post-pic est maximisée est ultérieur au point ou temps de mesure qui est égal à cVmax(k) lorsque k dépasse un point ou temps de fin de mesure sans détecter la Vmax.

8. Procédé selon l'une des revendications 1 à 7, comprenant en outre l'obtention de la réaction de coagulation jusqu'au point de mesure le plus récent sous la forme d'une réaction P(i).

9. Procédé selon la revendication 8, comprenant en outre le calcul d'un temps de coagulation sanguine de l'échantillon sanguin de sujet sur la base de P(i) ou V(i).

10. Procédé selon la revendication 9, dans lequel i > k₁ est satisfait.
